# EUROPEAN PATENT APPLICATION

(11) **EP 0 521 434 A1**
(43) Date of publication of application: **07.01.1993**
(21) Application number: 92110989.8
(22) Date of filing: 29.06.1992
(51) Int. Cl.: B05C 17/005, B65D 81/32, A61C 9/00

(54) **Modular fluid dosing device**

(30) Priority: 05.07.1991 IT PD910064 U
(71) Applicant: ZHERMACK S.r.L., I-45021 Badia Polesine (Rovigo) (IT)
(72) Inventor: Busin, Tiziano, I-45021 BADIA POLESINE(PROV. OF ROVIGO) (IT)
(74) Representative: Modiano, Guido, Dr.-Ing.

(57) **Abstract**

The modular fluid dosing device particularly for dosing pasty multiple-component products includes at least two separate tubular hollow bodies (1,2) and elements for coupling them mutually side by side. Each tubular body has a closure bottom (3,4), which is provided with an element (5,6) for the delivery of a corresponding component, and a sliding internal piston.

## Description

The present invention relates to a modular fluid dosing device, and in particular to a device for dosing pasty multiple-component products.

Products such as resins, adhesives, high-power adhesives, pastes for dental-mechanics use and the like are currently used which are formed by thoroughly mixing two components, generally a base and an activator.

A setting reaction generally occurs upon mixing in more or less long times.

Currently, the two components, in particular in the dental-mechanics field, are packaged, for their subsequent distribution and most of all in order to improve and facilitate their use, in double syringes, i.e. syringes which have a pair of cylindrical cavities, one for the base and one for the activator, arranged side by side.

The syringes are provided, at their bottom ends, with a delivery element which is connected to both cavities and to which a mixing and distribution tip is connectable.

However, double syringes made of plastics are affected by a severe problem which is due to the fact that the wall which divides the two cavities, in particular in the region adjacent to the inlet into the delivery element, is very thin, generally less than one millimeter thick, due to technical and manufacturing requirements.

Some of the products used are so aggressive and active that even during relatively short storage periods they are able to pass through the porosities of the dividing wall and contaminate one another.

This contamination usually occurs with the base setting reaction within periods of even only one or two months.

This problem creates severe difficulties both for the users, because they very often find that the product has deteriorated, and for the manufacturers, because the users consequently reject these otherwise very handy and practical devices.

Very often, manufacturers are forced to replace the product, and this naturally entails considerable problems in service costs and in corporate image.

The aim of the present invention is to provide a modular device particularly for the dosage of fluid and/or pasty multiple-component products which eliminates the problem described above in the known art.

Within the scope of the above aim, an object of the present invention is to provide a device which is easy to assemble for use.

Not least object is to provide a device which can be manufactured at low cost with conventional equipment and production facilities.

This aim, these objects and others which will become apparent hereinafter are achieved by a modular fluid dosing device, particularly for dosing pasty multiple-component products, characterized in that it comprises at least two separate tubular hollow bodies, each body being provided with a closure bottom which has a delivery element for a corresponding component and with an internal sliding piston, and means for coupling said hollow bodies side by side.

Further characteristics and advantages of the invention will become apparent from the detailed description of a preferred embodiment of the device, given by way of non-limitative example and illustrated in the accompanying drawings, wherein:
figure 1 is an exploded perspective view of the device according to the invention, with a mixing tip;
figure 2 is a perspective view of the device of figure 1, assembled with the mixing tip and a dispenser;
figure 3 is a perspective view of the device of figure 1 when assembled, in the region for coupling to the mixing tip;
figure 4 is a front elevation view of the parts which compose the device of figure 1, arranged side by side for coupling;

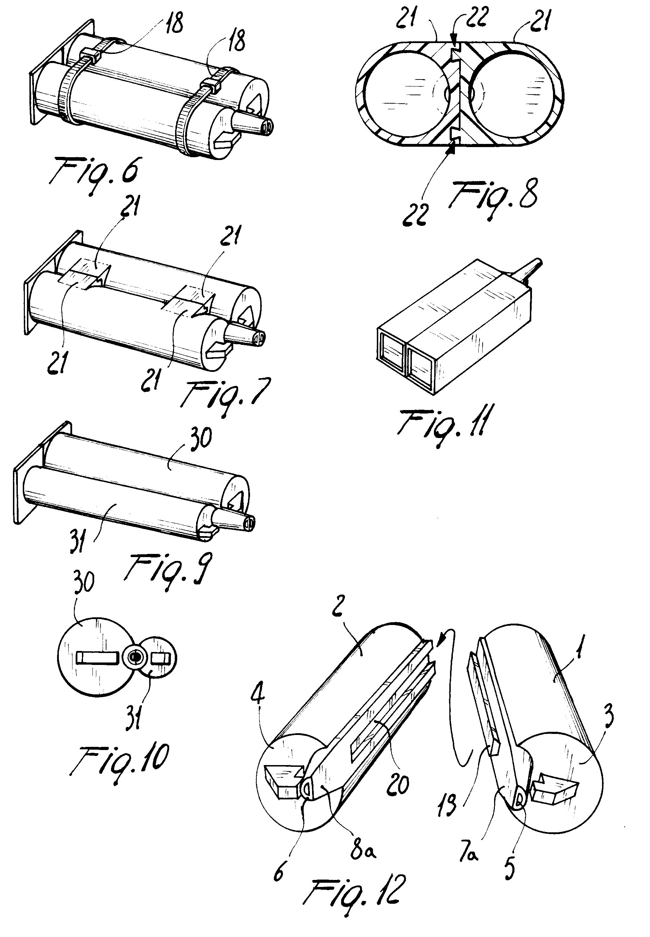

figure 5 is a longitudinal sectional view of a detail of the device of figure 1, with parts coupled and associated with the mixing tip;
figure 6 is a perspective view of a further aspect of the device, with parts coupled by means of straps;
figure 7 is a perspective view of a further aspect of the device, with parts assembled by joining protrusions which extend from the parts, laterally with respect to the coupling region;
figure 8 is a sectional view of a coupling with dovetail joints;
figure 9 is a perspective view of a further aspect of the device;
figure 10 is a front view of the device of figure 9;
figure 11 is a perspective view of a further aspect of the device;
figure 12 is a perspective view of a further type of coupling between the parts and the device.

With reference to the above figures, a modular fluid dosing device, particularly for the dosage of pasty multiple-component products and which is suitable for the dosage of binary products, comprises a pair of distinct tubular hollow bodies 1 and 2, each of which has a corresponding closure bottom 3 and 4 with a respective element 5 and 6 for the delivery of a component introduced during packaging in said tubular hollow body.

The delivery elements 5 and 6 extend with a respective planar region 7 and 8 for mutual coupling between the hollow bodies 1 and 2, which have corresponding sliding pistons 11 and 12 inserted therein from the open end, respectively 9 and 10.

The two delivery elements 5 and 6 are counter-shaped so as to form, when coupled side by side, a substantially cylindrical or tapered wing to be inserted in a known mixing tip 13.

Proximate to each delivery element 5 and 6 there are coupling means for the mixer 13 which comprise opposite protrusions, respectively designated by the reference numerals 14 and 15, which extend from the corresponding bottoms 3 and 4.

The protrusions 14 and 15 are suitable to engage, with a bayonet-like coupling, by means of their respective cantilever extensions 14a and 15a, a substantially oval flange 16 with which the mixing tip 13 is provided at its base.

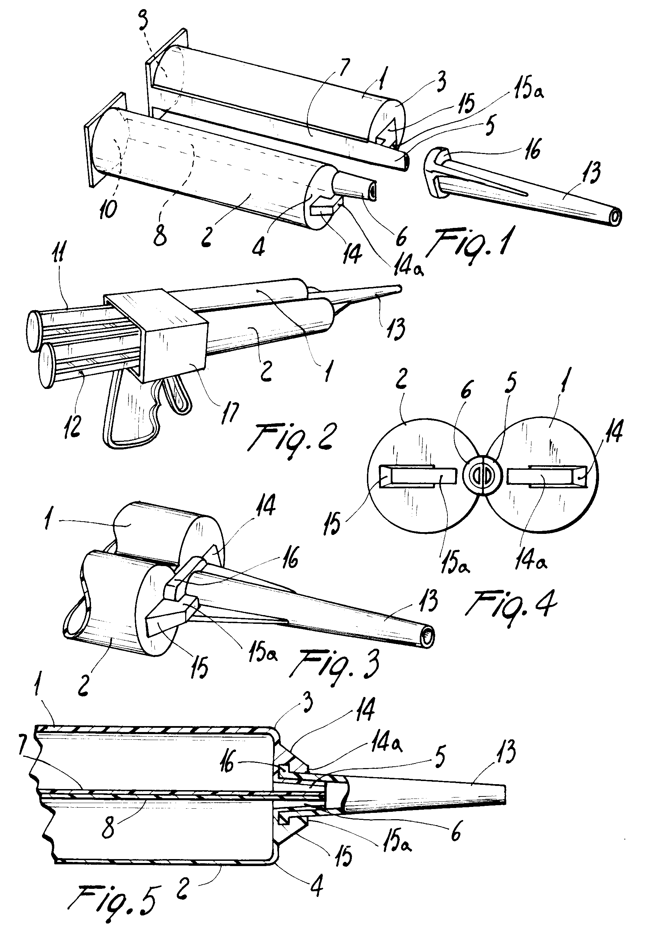

The two bodies 1 and 2 are coupled not only by the mixing tip 13 but also by a dispenser 17 of a per se known type, illustrated in figure 2, or by other manual, automatic or semiautomatic devices which are not illustrated.

The dispenser is provided with known means for simultaneously sliding said pistons in the hollow bodies which comprise a trigger actuated device which engages said pistons.

The coupling means can furthermore comprise, in a variated aspect, at least one strap 18, as shown in figure 6, which joins the hollow bodies 1 and 2 arranged side by side.

Also, the coupling means can comprise one or more dovetail joints in which male elements 19 couple to female seats 20 which are mutually counter-shaped.

The dovetail joints can be provided both directly on the walls to be arranged side by side upon coupling (7a and 8a in figure 12) and on protrusions 21 which extend from the lateral surface of each hollow body.

In this case, illustrated in figures 7 and 8, the joints are generally designated by 22.

According to the requirements of use, the various systems may naturally be variously combined in order to improve the coupling of the hollow bodies used.

The tubular hollow bodies 1 and 2 can have a substantially circular or oval cross-section with a respective flat coupling region 7 and 8 or, as shown in figure 11, can have a substantially polygonal, for example square, transverse cross-section.

The hollow bodies may furthermore have the same dimensions in transverse cross-section, or different ones, such as the bodies shown in figures 9 and 10.

In this case, the hollow body 30 has a greater diameter than the hollow body 31, if the dosage of the two components requires different proportions for mixing.

The operation of the device in the configuration of figure 2 is as follows: once the hollow bodies 1 and 2 have been arranged side by side with the regions 7 and 8 in contact and so that the delivery elements 5 and 6 form a single substantially cylindrical wing, the mixing tip 13 is inserted and the dispenser 17 is applied.

By acting on the dispenser 17, the pistons 11 and 12 are pushed simultaneously and both components exit into the mixing nozzle 13 through the dispensers 5 and 6.

In practice it has been observed that the device according to the invention has achieved the intended aim and objects, since in this manner two or more hollow bodies can be packaged independently of one another and subsequently coupled simply and rapidly for use.

The risk of contamination between the components, which currently arises when they are packaged in current double syringes in which they are divided by a thin partition, is thus eliminated.

Therefore, the problem due to the occurrence of setting of the components even for short periods of storage in the warehouse prior to sale, in laboratories and during use no longer exists.

Naturally, the two hollow bodies can also be easily disengaged if their content is not used up entirely at the same time.

It should also be stressed that the device according to the invention can be manufactured with plastics without particular problems, for example by injection-molding, and can thus be produced in a very large number of units at low cost.

In practice, the materials employed, so long as they are compatible with the contingent use, as well as the dimensions, may be any according to the requirements.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Modular fluid dosing device, particularly for dosing pasty multiple-component products, characterized in that it comprises at least two separate tubular hollow bodies (1,2), each of said bodies being provided with a respective closure bottom (3,4) which has a delivery element (5,6) for a corresponding component and with an internal sliding piston (11,12), the device further comprising means for coupling said hollow bodies side by side.

2. Device according to claim 1, characterized in that said means for the coupling of said hollow bodies comprise a mixing tip (13) in which said delivery elements are inserted.

3. Device according to claims 1 and 2, characterized in that said means for the coupling of said hollow bodies comprise bayonet-like couplings (14a,15a,16) which are suitable to associate said tip to the bottoms of said tubular hollow bodies.

4. Device according to claims 1 and 2, characterized in that said means for the coupling of said hollow bodies comprise at least one annular strap (18) which is suitable to be arranged so as to surround said hollow bodies arranged side by side.

5. Device according to claims 1 and 2, characterized in that said means for the coupling of said hollow bodies comprise dovetail joints (19,20), each component of which extends from a respective said hollow body.

6. Device according to claims 1 and 2, characterized in that said means for the coupling of said hollow bodies are constituted by male-female joints, each component of which extends from a respective said hollow body.

7. Device according to one or more of the preceding claims, characterized in that each tubular hollow body has a substantially cylindrical or oval cross-section and at least one flat region (7) for coupling to a corresponding flat region (8) of the complementary tubular hollow body.

8. Device according to one or more of claims 1 to 6, characterized in that each tubular hollow body has a substantially polygonal cross-section.

9. Device according to one or more of the preceding claims, characterized in that said tubular hollow bodies (30,31) have transverse cross-sections with different dimensions.
